# EUROPEAN PATENT SPECIFICATION

(11) **EP 1 518 583 B1**
(45) Date of publication and mention of the grant of the patent: **02.11.2006**
(21) Application number: 04021534.5
(22) Date of filing: 10.09.2004
(51) Int. Cl.: A61M 37/00, A61M 31/00, A61K 9/00

(54) **Injectable active micro-tanks for medical substances**
Injizierbarer, wirksamer Tank für medizinische Substanzen
Réservoir injectable actif pour des substances médicales

(30) Priority: 23.09.2003 IT TO20030728
(43) Date of publication of application: 30.03.2005
(73) Proprietor: C.R.F. Società Consortile per Azioni, 10043 Orbassano (TO) (IT)
(72) Inventor: Pizzi, Marco, 10100 Torino (IT); Grasso, Valentina, 10041 Carignano (Torino) (IT)
(74) Representative: Notaro, Giancarlo

(56) References cited:
- US-A- 3 608 549
- US-A- 4 507 115
- US-A- 4 793 825
- US-A- 5 391 164

## Description

The present invention relates to a device that can be injected into the bloodstream of a human or animal body, comprising at least one micro-tank, with dimensions not exceeding 10 µm, containing a medicinal substance, able to go from a closed configuration to an open configuration with consequent release of the medicinal as a result of an input signal coming from a source outside the human or animal body.

An intravascular device of the type indicated above is described and illustrated in the European Patent 0 178 769 B1 and in the US Patent US-A-4 793 825.

Medical research has long highlighted the need for greater selectivity in the administration of drugs. In particular in tumour therapy it would be very useful specifically to hit diseased cells whilst preventing collateral damage to healthy ones. To this end, two main research fields, based respectively on carrier micro-particles that chemically recognise the diseased cells, bringing the drug in contact with them, and carrier micro-particles with externally activated release. The present invention relates, in general, to this second type of solutions.

As indicated above, in the prior art a micro-tank has already been proposed with dimensions in the order of a few micrometres, injectable into the bloodstream and activated by means of an input signal originating from a source outside the human body, in order to release the drug in proximity to the diseased cells.

The above identified prior art solution, however, is characterised in that it is constituted by a semiconductor device whose structure is relatively complex.

The object of the present invention is to provide an intravascular device of the type set out above that has characteristics of high structural simplicity and operational reliability.

The main characteristic of the invention resides in the fact that the aforesaid micro-tank is defined by at least one film, capable of going from the aforesaid closed configuration and the aforesaid open configuration as a result of a temperature rise caused by said input signal.

Said result can be achieved for example by providing a bimetallic film constituted by two mutually coupled layers of metals having markedly different thermal expansion characteristics. In the resting condition, the film can for example have a furled cylindrical configuration and it unfurls instead in a plane as a result of a temperature rise that causes different thermal expansions of the two layers. Alternatively, one of the two layers can be constituted by a shape memory metal alloy that tends to go from the rolled configuration to the planar configuration when a transition temperature is exceeded.

Naturally, the closed configuration of the film can be any. For example, it is possible to think of a micro-tank with spherical shape, constituted by a series of wedges joined only in proximity to a pole of the sphere, which open in flower-like fashion and extend in a plane as a result of the temperature rise.

The metal constituting the internal layer of the film containing the micro-tank is a metal suitable to be "functionalised", i.e. able to allow to bind thereto the active ingredient constituting the drug. A particularly well suited metal for this purpose is gold. Therefore, it is possible to think for example of a film constituted by an inner layer of gold and by an outer layer of titanium, although obviously any other suitable solution is possible, for example selected among the pairs Au/SiO₂, Au/SiOₓ, Ti/SiOₓ, Tᵢ/Si, Au/Si. The thickness of each of the two layers is preferably in the order of tens of nanometres.

The input signal that generates the temperature rise needed to "open" the micro-tank or the micro-tanks injected into the bloodstream can be constituted by an electromagnetic radiation transmitted locally against the area of the body containing the diseased cells. The electromagnetic waves applied externally heat the micro-tanks because of the currents induced in the metal films, but without heating body tissues.

The temperature rise has two effects: causing the "opening" of the micro-tanks and causing the breaking of the chemical bonds between the medicinal substance and the inner layer of the micro-tanks. The medicinal substance is thus released in proximity to the diseased cells.

Additional characteristics and advantages of the present invention shall become readily apparent from the description that follows with reference to the accompanying drawings, provided purely by way of non limiting example, in which:
- Figure 1 is a section view of a first embodiment of a micro-tank according to the invention, in its closed configuration,
- Figure 2 is a section view of the micro-tank of Figure 1 in the open configuration,
- Figure 3 is a schematic view showing the operating principle of the invention,
- Figures 4,5 show a variant of micro-tank respectively in its closed configuration and in its open configuration.

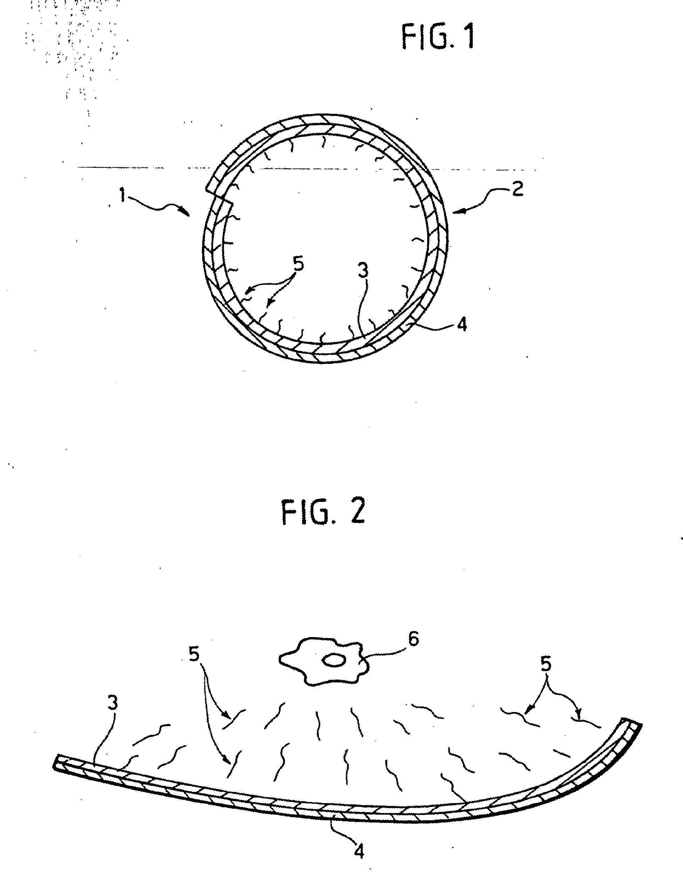

In Figure 1, the reference number 1 globally designates a micro-tanks for a medicinal substance, injectable into the bloodstream of a human or animal body. The outer diameter of said micro-tank can for example measure between 1 and 5 µm and it is generally smaller than 10 µm. The micro-tank is constituted by a rolled film 2 including an inner layer 3 of gold and an outer layer 4 of titanium, each having a thickness in the order of a few tens of micrometres. As stated, instead of the gold/titanium pair it is possible to use any other pair of suitable metals. There are two requirements to meet in the case of the illustrated example: first of all the metals constituting the two layers must have sufficiently different thermal expansion characteristics to assure the "opening" of the micro-tank as a result of a temperature rise; secondly, the material constituting the inner layer must be able to be "functionalised", i.e. it must allow to bind thereon the active ingredient constituting the medicinal substance.

Any one of the alternative solutions indicated above with reference to the metals constituting the two layers is usable, and in fact yet other materials can be selected, provided the aforesaid conditions are met.

Moreover, alternatively to the use of a bi-metallic film, it is also possible to use a film comprising at least one layer constituted by a shape memory metal alloy which is able to change its configuration upon exceeding a transition temperature.

Figures 1, 2 show how as a result of the opening of the micro-tank 1 due to a temperature rise the bonds between the gold layer and the active ingredient 5 are broken, thereby allowing the active ingredient 5 locally to attack the diseased cells 6.

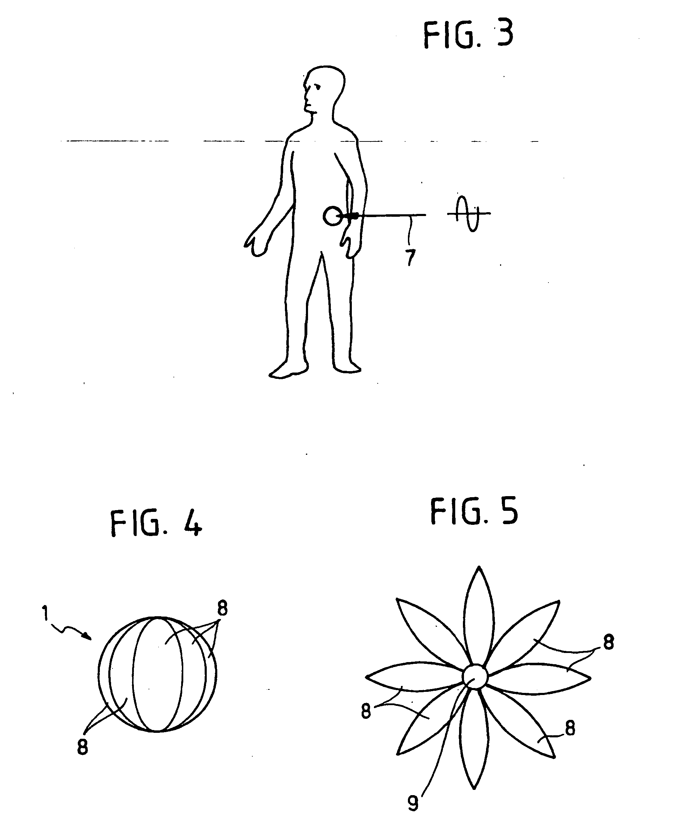

The temperature rise that causes the opening of the micro-tanks is obtained, as indicated previously, by heating with an electromagnetic radiation 7 (Figure 3) the area of the body where the cells to be treated are located. The temperature rise is due to the currents induced in the metals constituting the micro-tanks and therefore does not involve the body tissues.

Figures 4, 5 show a variant in which the micro-tank 1 has a spherical configuration and is constituted by a plurality of wedges 8 which are mutually joined only at a pole 9 of the sphere, so they are able to extend into the plane and open in flower-like fashion as shown in Figure 5, as a result of the temperature rise.

In a preferred embodiment the micro-tank is made of ferromagnetic material and magnetic fields applied from the exterior are used to confine the micro-tank in the diseased area, after injecting it in proximity to said area.

The magnetic characteristics of the micro-tank can also be used to filter the blood outside the body after a treatment, to free it from possible aggregation centres.

Naturally, without altering the principle of the invention, the construction details and the embodiments may vary widely from what is described and illustrated purely by way of example herein, without thereby departing from the scope of the present invention as defined in the appended claims.

## Claims

1. An intravascular device that can be injected into the bloodstream of a human or animal body, comprising at least one micro-tank, with dimensions not exceeding 10 µm, containing a medicinal substance (5), and able to go from a closed configuration to an open configuration with consequent release of the medicinal as a result of an input signal coming from a source outside the human or animal body,
**characterised in that** said micro-tank (1) is defined by at least one film (2), capable of going from the aforesaid closed configuration to the aforesaid open configuration as a result of a temperature rise caused by said input signal.

2. Intravascular device as claimed in claim 1, **characterised in that** said film is a bi-metallic film constituted by two mutually coupled layers of metals able to go from said closed configuration to said open configuration due to the different thermal expansion characteristics of the metals that constitute it.

3. Intravascular device as claimed in claim 1, **characterised in that** said film has an inner surface (3) whereto is bound said medicinal substance and which is able to release said medicinal substance as a result of said temperature rise.

4. Intravascular device as claimed in claim 1, **characterised in that** said film comprises at least one layer of shape memory material.

5. Intravascular device as claimed in claim 1, **characterised in that** in its closed configuration said micro-tank has a dimension of between 1 and 5 µm.

6. Intravascular device as claimed in claim 2, **characterised in that** said bimetallic film is constituted by a pair of materials chosen among Au/Ti, Au/SiO₂, Au/SiOₓ, Ti/SiOₓ,Tᵢ/Si, Au/Si.

7. Intravascular device as claimed in claim 2, **characterised in that** the thickness of each layer of the film is in the order of a few tens of µm.

8. Intravascular device as claimed in claim 1, **characterised in that** thereto are associated means for generating said input signal in the form of an electromagnetic radiation, in such a way as to cause said temperature rise as a result of the electrical currents induced by said electromagnetic radiation in the materials constituting the aforesaid film.

9. Intravascular device as claimed in claim 1, **characterised in that** the micro-tank includes a ferromagnetic material and that to said device are associated means for applying a magnetic field from the exterior to confine the micro-tank in a specific area of the body.

10. Pharmaceutical preparation comprising a plurality of intravascular devices as claimed in any of the previous claims, in a pharmaceutically acceptable carrier substance.

11. A device for administering a medicinal substance, comprising at least one intravascular device as claimed in any of the claims 1-9 and means for directing an electromagnetic radiation from a source outside the human or animal body towards an area of said body, to cause a rise in the temperature of said micro-tank as a result of the electrical currents induced in the film that constitutes it, without substantially altering the temperature of the body tissues.

## Patentansprüche

1. Intravaskuläre Vorrichtung, die in einen Blutstrom eines menschlichen oder tierischen Körpers injiziert werden kann und wenigstens einen Mikrobehälter umfasst, der Abmessungen, die 10 µm nicht überschreiten, aufweist und eine medizinische Substanz (5) enthält und als Folge eines Eingangssignals, das von einer Quelle außerhalb des menschlichen oder tierischen Körpers kommt, von einer geschlossenen Konfiguration zu einer offenen Konfiguration mit darauffolgender Freisetzung der medizinischen Substanz übergehen kann,
**dadurch gekennzeichnet, dass** der Mikrobehälter (1) durch wenigstens eine Folie (2) gebildet wird, die als Folge eines von dem Eingangssignal verursachten Temperaturanstiegs von der vorgenannten geschlossenen Konfiguration zu der vorgenannten offenen Konfiguration übergehen kann.

2. Intravaskuläre Vorrichtung nach Anspruch 1, **dadurch gekennzeichnet, dass** die Folie eine Bimetallfolie ist, die von zwei wechselseitig verbundenen Schichten aus Metallen gebildet wird und auf Grund der unterschiedlichen Wärmedehnungseigenschaften der Metalle, die sie bilden, von der geschlossenen Konfiguration zu der offenen Konfiguration übergehen kann.

3. Intravaskuläre Vorrichtung nach Anspruch 1, **dadurch gekennzeichnet, dass** die Folie eine Innenfläche (3) aufweist, an die die medizinische Substanz gebunden ist und die als eine Folge des Temperaturanstiegs die medizinische Substanz freisetzen kann.

4. Intravaskuläre Vorrichtung nach Anspruch 1, **dadurch gekennzeichnet, dass** die Folie wenigstens eine Schicht aus Formgedächtnismaterial umfasst.

5. Intravaskuläre Vorrichtung nach Anspruch 1, **dadurch gekennzeichnet, dass** der Mikrobehälter in seiner geschlossenen Konfiguration eine Abmessung zwischen 1 und 5 µm aufweist.

6. Intravaskuläre Vorrichtung nach Anspruch 2, **dadurch gekennzeichnet, dass** die Bimetallfolie durch ein Paar aus Materialien, die aus Au/Ti, Au/SiO₂, Au/SiOₓ, Ti/SiOₓ, Ti/Si, Au/Si gewählt wurden, gebildet wird.

7. Intravaskuläre Vorrichtung nach Anspruch 2, **dadurch gekennzeichnet, dass** die Dicke jeder Schicht der Folie in der Größenordnung weniger zehntel µm liegt.

8. Intravaskuläre Vorrichtung nach Anspruch 1, **dadurch gekennzeichnet, dass** Einrichtungen zum Erzeugen des Eingangssignals in der Form einer elektromagnetischen Strahlung so damit assoziiert sind, dass der Temperaturanstieg als Folge der elektrischen Ströme verursacht wird, die durch die elektromagnetische Strahlung bei den Materialien, die die vorgenannte Folie bilden, induziert werden.

9. Intravaskuläre Vorrichtung nach Anspruch 1, **dadurch gekennzeichnet, dass** der Mikrobehälter ein ferromagnetisches Material umfasst und dass mit der Vorrichtung Einrichtungen zum Anwenden eines Magnetfelds von außen assoziiert sind, um den Mikrobehälter in einem spezifischen Bereich des Körpers einzugrenzen.

10. Pharmazeutisches Präparat, das eine Vielzahl intravaskulärer Vorrichtungen nach einem der vorhergehenden Ansprüche umfasst, in einer pharmazeutisch akzeptablen Trägersubstanz.

11. Vorrichtung zum Verabreichen einer medizinischen Substanz, umfassend wenigstens eine intravaskuläre Vorrichtung nach einem der Ansprüche 1 bis 9 und Einrichtungen zum Richten einer elektromagnetischen Strahlung von einer Quelle außerhalb des menschlichen oder tierischen Körpers zu einem Bereich des Körpers hin, um einen Anstieg der Temperatur des Mikrobehälters als Folge der elektrischen Ströme, die in der ihn bildenden Folie induziert werden, zu verursachen, ohne die Temperatur der Körpergewebe im Wesentlichen zu verändern.

## Revendications

1. Dispositif intravasculaire qui peut être injecté dans la circulation sanguine d'un corps humain ou animal, comprenant au moins un microréservoir, dont les dimensions n'excèdent pas 10 µm, contenant une substance médicale (5) et capable de passer d'une configuration fermée à une configuration ouverte avec une libération consécutive de la substance médicale à la suite d'un signal d'entrée provenant d'une source située à l'extérieur du corps humain ou animal,
**caractérisé en ce que** ledit microréservoir (1) est défini par au moins un film (2) capable de passer de la configuration fermée précitée à la configuration ouverte précitée à la suite d'une élévation de la température provoquée par ledit signal d'entrée.

2. Dispositif intravasculaire selon la revendication 1, **caractérisé en ce que** ledit film est un film bimétallique, constitué de deux couches de métaux couplées mutuellement, capable de passer de ladite configuration fermée à ladite configuration ouverte en raison des différentes caractéristiques de dilatation thermique des métaux qui le constituent.

3. Dispositif intravasculaire selon la revendication 1, **caractérisé en ce que** ledit film possède une surface interne (3) à laquelle est liée ladite substance médicale et qui est capable de libérer ladite substance médicale à la suite de ladite élévation de température.

4. Dispositif intravasculaire selon la revendication 1, **caractérisé en ce que** ledit film comprend au moins une couche d'un matériau à mémoire de forme.

5. Dispositif intravasculaire selon la revendication 1, **caractérisé en ce que**, dans sa configuration fermée, ledit microréservoir a une dimension comprise entre 1 et 5 µm.

6. Dispositif intravasculaire selon la revendication 2, **caractérisé en ce que** ledit film bimétallique est constitué d'une paire de composés choisie parmi Au/Ti, Au/SiO₂, Au/SiOₓ, Ti/SiOₓ, Ti/Si et Au/Si.

7. Dispositif intravasculaire selon la revendication 2, **caractérisé en ce que** l'épaisseur de chaque couche du film est de l'ordre de quelques dizaines de µm.

8. Dispositif intravasculaire selon la revendication 1, **caractérisé en ce qu'**on lui associe un moyen pour générer ledit signal d'entrée sous la forme d'un rayonnement électromagnétique, de manière à provoquer ladite élévation de température à la suite des courants électriques induits par ledit rayonnement électromagnétique dans les matériaux constituant le film précité.

9. Dispositif intravasculaire selon la revendication 1, **caractérisé en ce que** le microréservoir comprend un composé ferromagnétique et **en ce que** l'on associe audit dispositif un moyen pour appliquer un champ magnétique depuis l'extérieur afin de confiner le microréservoir dans une zone spécifique du corps.

10. Préparation pharmaceutique comprenant une pluralité de dispositifs intravasculaires selon l'une quelconque des revendications précédentes, dans une substance véhiculaire acceptable au plan pharmaceutique.

11. Dispositif d'administration d'une substance médicale, comprenant au moins un dispositif intravasculaire selon l'une quelconque des revendications 1 à 9 et un moyen destiné à diriger un rayonnement électromagnétique (7) depuis une source externe au corps humain ou animal vers une zone dudit corps, afin de provoquer une élévation de température dudit microréservoir à la suite des courants électriques induits dans le film qui le constituent, sans pour autant sensiblement altérer la température des tissus corporels.
